# EUROPÄISCHE PATENTSCHRIFT

(11) **EP 3 164 706 B1**
(45) Veröffentlichungstag und Bekanntmachung des Hinweises auf die Patenterteilung: **31.01.2018**
(21) Anmeldenummer: 16708922.6
(22) Anmeldetag: 29.02.2016
(51) Int. Cl.: G01N 33/36

(54) **GARNPRÜFGERÄT MIT EINER GARNEINFÜHRUNGSVORRICHTUNG**
YARN-CHECKING DEVICE HAVING A YARN INSERTION DEVICE
APPAREIL DE CONTRÔLE DE FIL MUNI D'UN DISPOSITIF D'INTRODUCTION DE FIL

(30) Priorität: 20.03.2015 CH 4112015
(43) Veröffentlichungstag der Anmeldung: 10.05.2017
(73) Patentinhaber: Uster Technologies AG, 8610 Uster (CH)
(72) Erfinder: SCHEIBER, Patrik, CH-8155 Niederhasli (CH); KUSTER, Martin, CH-8733 Eschenbach (CH); DE VRIES, Loris, CH-8625 Gossau (CH); KOLLER, Beat, CH-8638 Goldingen (CH); FENNER, Jürg, CH-8600 Dübendorf (CH)
(74) Vertreter: Pliska, Pavel
(86) Internationale Anmeldenummer: PCT/CH2016/000035
(87) Internationale Veröffentlichungsnummer: WO 2016/149841

(56) Entgegenhaltungen:
- EP-A2- 0 585 555
- WO-A1-2007/014475
- DE-A1- 2 041 042
- DE-A1- 3 544 615

## Beschreibung

### FACHGEBIET

Die vorliegende Erfindung liegt auf dem Gebiet der textilen Qualitätsprüfung und bezieht sich auf ein Garnprüfgerät zur Prüfung einer Garnprobe, gemäss dem Oberbegriff des ersten Patentanspruchs. Das erfindungsgemässe Garnprüfgerät ist als eigenständige, nicht in einen Produktionsprozess eingebundene Vorrichtung konzipiert und somit im Off-line-Betrieb, bspw. im Textillabor, einsetzbar.

### STAND DER TECHNIK

Garnprüfgeräte werden eingesetzt, um die Qualität von Garnen zu prüfen. Es geht darum, einerseits eine gleich bleibende Qualität der herzustellenden textilen Produkte zu erreichen, andererseits aber auch darum, den textilen Produktionsprozess anhand von Stichproben off-line zu überwachen.

Die bekannten Garnprüfgeräte dieser Art arbeiten nach dem Durchlaufprinzip, d. h. das zu prüfende Garn wird in einem einzigen Prüfdurchlauf von einer Garnspule abgezogen und durchläuft in serieller Weise im Garnprüfgerät eine Anordnung von Sensoren, um die zu bestimmenden Parameter zu messen. Dies ist grundsätzlich ein kontinuierlicher Prozess. Garnprüfgeräte neueren Typs können automatisch eine Mehrzahl von Garnproben nacheinander abarbeiten, die vorgängig von einer Bedienperson manuell eingelegt werden müssen. Der Probenwechsel, die Einführung der jeweiligen Garnprobe in einen Garnpfad und ihre Ausmessung erfolgen dann automatisch ohne Eingriff der Bedienperson.

Die US-2,590,398 A zeigt ein Garnprüfgerät mit einem aktiv betätigbaren Greifmechanismus, der auf einer geschlossenen Kette montiert ist. Die Kette läuft auf einer Bahn in Form eines Rechtecks, dessen eine Seite ungefähr mit dem Garnpfad zusammenfällt. Der mit der Kette umlaufende Greifmechanismus ergreift die Garnprobe am Anfang des Garnpfades, führt sie in den Garnpfad ein und lässt sie am Ende des Garnpfades los.

Ein Garnprüfgerät mit einem halbautomatischen Garnwechsler und einer automatischen Garneinführungsvorrichtung ist in der US-3,805,607 A und in der DE2041042 A1 offenbart. Eine Mehrzahl von zu prüfenden Garnproben wird manuell in den Garnwechsler eingelegt. Im Prüfbetrieb ist die Position des Garnwechslers automatisch auf den Garnpfad und die Garneinführungsvorrichtung ausrichtbar. Die sich jeweils in einer Prüfposition befindliche Garnprobe wird von der Garneinführungsvorrichtung ergriffen und automatisch in einen Garnpfad des Prüfgerätes eingelegt, worauf die Garnprüfung beginnen kann. Die Garneinführungsvorrichtung ist als ortsfest gelagerter Schwenkarm ausgebildet, an dessen freiem Ende ein aktiv betätigbarer Greifmechanismus montiert ist. Der Schwenkarm vollführt beim Einlegen des Garns eine Drehbewegung von ca. 90°. Eine ähnliche Garneinführungsvorrichtung wie die US-3,805,607 A zeigt auch die US-5,351,535 A. Bei der letzteren vollführt aber der Schwenkarm beim Einlegen des Garns eine Drehbewegung von ca. 180°. Ein Nachteil der Garneinführungsvorrichtungen mit Schwenkarm ist der grosse Kreisbogen, auf welchem der Schwenkarm und der Greifmechanismus weit über eine Front des Gamprüfgerätes hinaus rotieren. Dies bringt die Gefahr von Verletzungen für Bedienpersonen mit sich.

Die US-2008/0209998 A1 zeigt ein anderes Garnprüfgerät mit einem halbautomatischen Garnwechsler und einer automatischen Garneinführungsvorrichtung, nämlich das Garnprüfgerät USTER® *TESTER 5* der Anmelderin des vorliegenden Schutzrechtes. Die Garneinführungsvorrichtung des USTER® *TESTER* 5 vollführt keine Dreh-, sondern eine im Wesentlichen (bis auf eine leichte Krümmung) lineare Verschiebungsbewegung. Somit verringert sie die Verletzungsgefahr für Bedienpersonen erheblich. Der verschiebbare Greifer ist als zweizinkige Gabel ausgebildet und funktioniert passiv. Voraussetzung für den Einsatz einer solchen Garneinführungsvorrichtung ist, dass der Garnwechsler praktisch bündig mit einer Front des Gamprüfgerätes angebracht ist. Eine solche Anordnung hat wiederum den Nachteil, dass der Garnwechsler nur umständlich und unter grossem Zeitaufwand beschickt werden kann. Die Bedienperson muss zuerst alle Garnproben von der Rückseite her lose in der richtigen Reihenfolge auf dem Garnwechsler bereitlegen.

Danach geht sie um das Garnprüfgerät herum zu seiner Vorderseite und klemmt jedes der bereitliegenden Garnenden in entsprechende Garnklemmen ein. Die DE3544615 A1 offenbart eine Garneinführungsvorrichtung mit translatorischer und Rotationsbewegung.

### DARSTELLUNG DER ERFINDUNG

Es ist eine Aufgabe der vorliegenden Erfindung, Garneinführungsvorrichtungen in Garnprüfgeräten, wie sie aus dem Stand der Technik bekannt sind, weiter zu verbessern. Insbesondere soll die Verletzungsgefahr für Bedienpersonen durch die Garnreinführungsvorrichtung klein sein. Die Garneinführungsvorrichtung soll mehr Möglichkeiten für das Handling der Garnprobe bieten als die bekannten Garneinführungsvorrichtungen. Das Ergreifen der Garnprobe und die Garneinführung sollen mindestens so zuverlässig oder zuverlässiger sein wie bei den Garnwechselvorrichtungen gemäss dem Stand der Technik. Die Garneinführungsvorrichtung soll an eine halbautomatische Garnwechselvorrichtung angepasst sein, bei welcher das manuelle Einlegen der Garnproben besonders einfach und zeitsparend ist.

Diese und andere Aufgaben werden durch das im ersten Patentanspruch definierte Garnprüfgerät gelöst. Vorteilhafte Ausführungsformen sind in den abhängigen Patentansprüchen angegeben.

Der Erfindung liegt die Idee zugrunde, eine Greifeinrichtung der Garneinführungsvorrichtung sowohl verschiebbar als auch drehbar zu gestalten, wobei die Verschiebbarkeit und die Drehbarkeit unabhängig voneinander sind. Die Verschiebung erfolgt im Wesentlichen parallel zum Garnpfad und die Drehung um eine Drehachse, die senkrecht zum Garnpfad und parallel zu einer durch die Front definierten Frontebene liegt. Die Begriffe "ergreifen", "Greifeinrichtung" u. ä. werden in dieser Schrift für jegliche Wechselwirkung mit dem Garn zwecks seiner Einführung in den Garnpfad verwendet. Der Begriff "Greifeinrichtung" umfasst also sowohl passive Einrichtungen wie die aus dem USTER® *TESTER* 5 bekannte Gabel als auch aktive Einrichtungen wie den aus der US-2,590,398 A bekannten Greifmechanismus.

Das erfindungsgemässe Garnprüfgerät zur Prüfung einer Garnprobe beinhaltet einen entlang einer Front des Gamprüfgerätes verlaufenden Garnpfad, mindestens einen entlang des Garnpfades angeordneten Sensor zur Prüfung der Garnprobe und eine Garneinführungsvorrichtung zur Einführung der Garnprobe in den Garnpfad. Die Garneinführungsvorrichtung weist eine bewegliche Greifeinrichtung zum reversiblen Ergreifen der Garnprobe auf. Die Greifeinrichtung ist einerseits in einer Richtung, die im Wesentlichen parallel zum Garnpfad liegt, verschiebbar und andererseits um eine Drehachse, die senkrecht zum Garnpfad und parallel zu einer durch die Front definierten Frontebene liegt, drehbar. Die Verschiebbarkeit und die Drehbarkeit sind unabhängig voneinander.

In einer Ausführungsform beinhaltet die Garneinführungsvorrichtung einen verschiebbaren Wagen, auf dem ein Greifarm drehbar gelagert ist, an dessen freiem Ende sich die Greifeinrichtung befindet. Der Wagen ist vorzugsweise linear verschiebbar. Der Wagen kann von einem im Garnprüfgerät befestigten ersten Motor über mindestens einen Treibriemen zur Verschiebung antreibbar sein. Der Greifarm kann von einem auf dem Wagen befestigten zweiten Motor zur Drehung antreibbar sein. Der Greifarm ist vorzugsweise derart ausgebildet, dass die Greifeinrichtung einerseits in axialer Richtung vom Wagen und andererseits in radialer Richtung von der Drehachse beabstandet ist.

In einer Ausführungsform ist die Greifeinrichtung pneumatisch betätigbar. Der Greifarm kann als Rohr ausgebildet sein, durch welches Druckluft der Greifeinrichtung zuführbar ist.

In einer Ausführungsform weist die Greifeinrichtung ein Paar im Wesentlichen zueinander parallele, zusammenwirkende Klemmflächen, einen Schliessmechanismus zum Drücken der Klemmflächen gegeneinander und einen Öffnungsmechanismus zum Entfernen der Klemmflächen voneinander auf. Der Schliessmechanismus beinhaltet z. B. eine vorgespannte Feder, deren Federkraft die Klemmflächen gegeneinander drückt, und der Öffnungsmechanismus beinhaltet z. B. einen einfachwirkenden Pneumatikzylinder, bei dessen Beaufschlagung mit Druckluft die Klemmflächen voneinander entfernt werden.

In einer Ausführungsform weist das Garnprüfgerät eine halbautomatische Garnwechselvorrichtung zur Bereitstellung einer Mehrzahl von Garnproben auf. Die halbautomatische Garnwechselvorrichtung beinhaltet eine Einspanneinrichtung mit einer Mehrzahl von starr nebeneinander angeordneten, voneinander beabstandeten Einspannstellen zum Einspannen der Garnproben und eine Transporteinrichtung zum Transportieren der Einspanneinrichtung, wodurch eine zu prüfende, an irgendeiner der Einspannstellen eingespannte Garnprobe in eine vorgegebene Prüfposition transportierbar ist. Die Garnreinführungsvorrichtung ist derart ausgestaltet und bezüglich der Garnwechselvorrichtung angeordnet, dass eine in der Prüfposition eingespannte Garnprobe von ihrer Greifeinrichtung ergreifbar ist. Besonders vorteilhaft ist es, wenn jede Einspannstelle eine Garneinlauföse und zwei stromabwärts der Garneinlauföse angeordnete, fluchtende Garnklemmen beinhaltet, und die Garneinlauföse und die beiden Garnklemmen voneinander beabstandet im Wesentlichen auf einer Geraden liegen. Die halbautomatische Garnwechselvorrichtung kann auf einer Decke des Garnprüfgerätes angebracht und gegenüber der Front des Garnprüfgerätes um mindestens einen Drittel der Tiefe des Garnprüfgerätes nach hinten versetzt sein. Die Transporteinrichtung der halbautomatischen Garnwechselvorrichtung ist z. B. derart ausgestaltet, dass sie eine Linearverschiebung der Einspanneinrichtung bewirkt und die Richtung der Linearverschiebung senkrecht zum Garnpfad und parallel zu der Frontebene liegt.

Durch die Verschiebbarkeit und Drehbarkeit der Greifeinrichtung eröffnet die Garneinführungsvorrichtung neue Möglichkeiten für das Handling der Garnproben. So kann die Garneinführungsvorrichtung nicht nur zur Garneinführung, sondern auch zum Drücken der Garnprobe in eine Schneideinrichtung verwendet werden. Dank der grösseren Flexibilität ist auch die Zuverlässigkeit des Ergreifens der Garnprobe und der Garneinführung gewährleistet. Der Drehradius des Greifarms kann kleiner gehalten werden als bei Garneinführungsvorrichtungen, bei denen der Greifarm nur drehbar, aber nicht verschiebbar ist, woraus sich eine kleinere Verletzungsgefahr für Bedienpersonen ergibt. Das erfindungsgemässe Garnprüfgerät kombiniert vorzugsweise die Garneinführungsvorrichtung mit einer halbautomatischen Garnwechselvorrichtung, bei welcher das manuelle Einlegen der Garnproben besonders einfach und zeitsparend ist. Die Bedienperson kann jeweils ein Ende einer Garnprobe von einem hinter dem Garnprüfgerät befindlichen Garnwagen abziehen und mit einer einzigen Handbewegung in die Garnwechselvorrichtung einlegen. Wenn sie dies mit allen Garnproben getan hat, ist die Garnwechselvorrichtung fertig beschickt; weitere Manipulationen erübrigen sich. Dadurch spart die Bedienperson beim Beschicken der Garnwechselvorrichtung wertvolle Zeit, die sie für andere Tätigkeiten verwenden kann.

### AUFZÄHLUNG DER ZEICHNUNGEN

Nachfolgend wird eine Ausführungsform der Erfindung anhand der Zeichnungen detailliert erläutert.
- Figur 1: zeigt ein erfindungsgemässes Garnprüfgerät in einer perspektivischen Ansicht von links vorn.
- Figur 2: zeigt eine Garnwechselvorrichtung für das erfindungsgemässe Garnprüfgerät in einer perspektivischen Ansicht von oben rechts vorn.
- Figur 3: zeigt schematisch die gegenseitige Anordnung einer Garnwechselvorrichtung und einer Garneinführungsvorrichtung in einem erfindungsgemässen Garnprüfgerät.
- Figur 4: zeigt eine Garneinführungsvorrichtung für das erfindungsgemässe Garnprüfgerät in einer Seitenansicht.
- Figur 5: zeigt einen Teil der Garneinführungsvorrichtung von Figur 4 in einer perspektivischen Ansicht von links vorn.
- Figur 6: zeigt Längsschnitte durch eine Greifeinrichtung für die Garneinführungsvorrichtung von Figur 5 (a) in geschlossenem Zustand und (b) in offenem Zustand.

### AUSFÜHRUNG DER ERFINDUNG

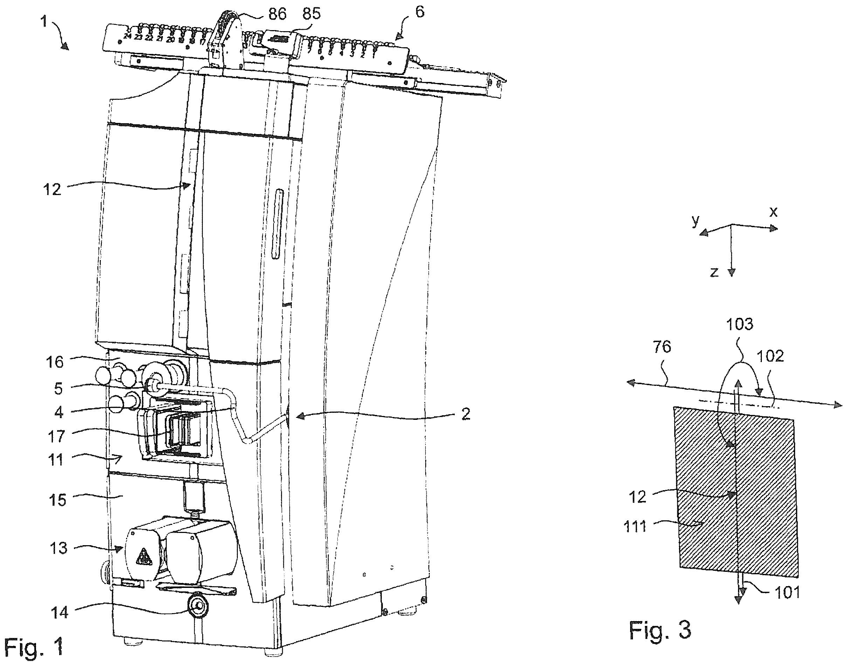

**Figur 1** zeigt beispielhaft eine Ausführungsform des erfindungsgemässen Garnprüfgerätes 1 in einer Ansicht von links vorn. Das Garnprüfgerät 1 weist eine Front 11 auf, entlang welcher ein Garnpfad 12 für eine zu prüfende (in Figur 1 nicht eingezeichnete) Garnprobe verläuft. Auf einer Seite des Garnprüfgerätes 1, welche von der Front 11 verschieden ist, ist eine automatische Garneinführungsvorrichtung 2 zum Einführen der Garnprobe in den Garnpfad 12 angebracht. Zum Einbringen in den Garnpfad 12 wird die Garnprobe von einem verschiebbaren und drehbaren Greifer 5 der Garneinführungsvorrichtung 2 ergriffen und durch eine entsprechende Bewegung des Greifers 5 eingebracht, worauf unten detailliert eingegangen wird.

Während des Prüfvorgangs tritt die Garnprobe durch eine erfindungsgemässe automatische Garnwechselvorrichtung 6 in den Garnpfad 12 ein. Im Garnpfad 12 durchläuft die Garnprobe verschiedene Sensoren. Die Garnprobe wird von einer Fördereinrichtung 13 entlang ihrer Längsrichtung durch den Garnpfad 12 gefördert. Schliesslich verlässt die Garnprobe den Garnpfad 12 durch eine Absaugöffnung 14.

Das Garnprüfgerät 1 hat einen turmartigen Aufbau. Der turmartige Aufbau kann verschiedene Funktionsmodule umfassen. Ein erstes Funktionsmodul 15 beinhaltet im vorliegenden Fall die Fördereinrichtung 13 und die Absaugöffnung 14. Ein zweites Funktionsmodul 16 beinhaltet eine kapazitive Sensorbaugruppe 17 zur Gleichmässigkeitsprüfung des Garns. Weitere, oberhalb des zweiten Funktionsmoduls 16 befindliche Funktionsmodule können weitere, z. B. optische Sensoren beinhalten, sind jedoch wegen einer Frontabdeckung in Figur 1 nicht sichtbar.

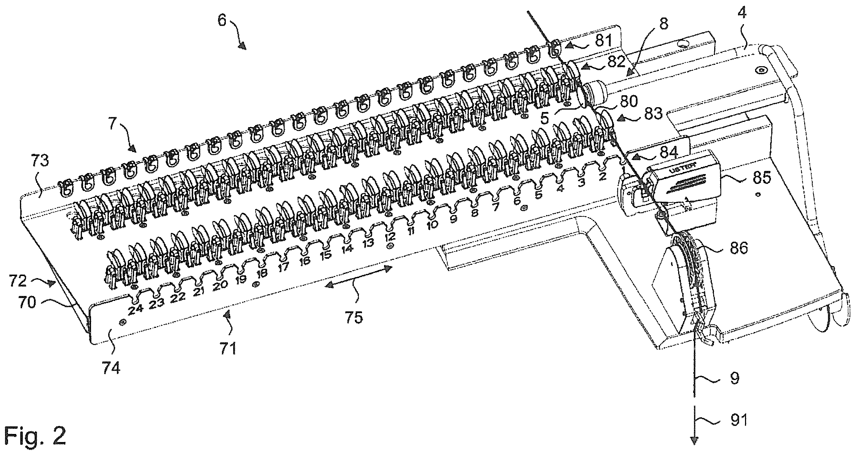

In **Figur 2** ist die Garnwechselvorrichtung 6 in einer Ansicht von oben rechts vorn dargestellt. Sie beinhaltet eine Einspanneinrichtung 7 mit einer Mehrzahl von starr nebeneinander angeordneten, voneinander beabstandeten Einspannstellen 8 zum Einspannen von Garnproben 9. Die hier diskutierte Ausführungsform hat 24 Einspannstellen 8, die äquidistant auf einer Länge von 455 mm angeordnet sind; die Äquidistanz beträgt somit 19.8 mm. Jede Einspannstelle 8 beinhaltet eine Garneinlauföse 81 und zwei stromabwärts der Garneinlauföse 81 angeordnete, fluchtende Garnklemmen 82, 83. Die Garneinlauföse 81 und die beiden Garnklemmen 82, 83 liegen voneinander beabstandet im Wesentlichen auf einer Geraden 80. Die beiden Garnklemmen 82, 83 dienen zum Einspannen der Garnprobe 9. Die Garneinlauföse 81 dient dazu, eine Grobposition für die Garnprobe 9 vorzugeben und eine erste Garnklemme 82 von Zugkräften quer zur Garnlängsrichtung zu entlasten. Ferner kann jede Einspannstelle 8 eine stromabwärts der beiden Garnklemmen 82, 83 angeordnete Garnauslauföse 84 beinhalten. Die Garnauslauföse 84 liegt aber vorzugsweise unterhalb der genannten Geraden 80 und soll nicht mit der laufenden Garnprobe 9 wechselwirken; sie dient vielmehr bloss dazu, im Ruhezustand das freie Ende einer in der Einspannstelle 8 eingespannten Garnprobe 9 in einer definierten Position zu halten.

In der hier diskutierten Ausführungsform sind die Einspannstellen 8 auf einer ebenen, im Wesentlichen rechteckigen Trägerplatte 70 angeordnet. Eine längere Seite 71 der Trägerplatte 70 verläuft horizontal. Eine kürzere Seite 72 der Trägerplatte 70 ist parallel zur genannten Geraden 80 und bspw. um 25° gegen die Horizontale so geneigt, dass die Garnlaufrichtung schräg von unten nach oben verläuft. Bei einer solchen Neigung liegen die Garnklemmen 82, 83 einer jeden Einspannstelle 8 höher als die Garneilauföse 81 derselben Einspannstelle 8. Die Einspannstellen 8 sind horizontal nebeneinander angeordnet, und alle Garneinlaufösen 81 liegen auf derselben Höhe. Die Neigung der Trägerplatte 70 gegen die Rückseite des Garnprüfgerätes 1 hin nach unten erleichtert das Einlegen der Garnproben 9 in die Einspanneinrichtung 8. Um von dieser Erleichterung Gebrauch zu machen, sollte der Neigungswinkel der Trägerplatte 70 gegen die Horizontale zwischen 0° und 45° betragen. Die Trägerplatte 70 kann auch horizontal liegen (Neigungswinkel 0°), sollte aber nicht nach vorn geneigt sein, weil dadurch das Einlegen erschwert würde. Die Trägerplatte 70 trägt die Garnklemmen 82, 83. Sie weist an ihren stromaufwärts und stromabwärts gelegenen Rändern aus ihrer Ebene heraus ragende Flansche 73, 74 auf, welche die Garneinlaufösen 81 bzw. die Garnauslaufösen 84 tragen.

In Figur 2 ist in einer ersten Einspannstelle 8 eine Garnprobe 9 eingezeichnet. Die Längsrichtung des in der Einspannstelle befindlichen Teils der Garnprobe 9 fällt mit der genannten Geraden 80 zusammen. Die Laufrichtung der Garnprobe 9 während ihrer Prüfung im Garnprüfgerät 1 ist durch einen Pfeil 91 angedeutet, auf welche Laufrichtung sich auch die in dieser Schrift verwendeten Begriffe "stromabwärts" und "stromaufwärts" beziehen.

Ferner beinhaltet die erfindungsgemässe Garnwechselvorrichtung 6 eine (in den Zeichnungen nicht sichtbare) Transporteinrichtung zum Transportieren der Einspanneinrichtung 7. In der hier diskutierten Ausführungsform handelt es sich beim Transport der Einspanneinrichtung 7 um eine Linearverschiebung. Die Verschiebungsrichtung ist durch einen Doppelpfeil 75 angedeutet. Sie ist horizontal, parallel zur längeren Seite 71 der Trägerplatte 70 und senkrecht zur genannten Geraden 80. Das Transportieren der Einspanneinrichtung 7 dient dazu, eine zu prüfende, an irgendeiner der Einspannstellen 8 eingespannte Garnprobe 9 in eine Prüfposition zu bringen. Die Prüfposition ist durch die Lage des Garnpfades 12 im Garnprüfgerät 1 vorgegeben. Die in Figur 2 eingezeichnete Garnprobe 9 befindet sich gerade in der Prüfposition. Ein mit dem Garnpfad 12 fluchtendes Umlenkrad 86 kann die in der Prüfposition befindliche Garnprobe 9 zum Garnpfad 12 hin umlenken. Zwischen der Einspanneinrichtung 7 und dem Umlenkrad 86 kann sich eine Schneideinrichtung 85 zum Durchtrennen der Garnprobe 9 nach erfolgter Prüfung befinden.

In Figur 2 ist auch der Greifer 5 der Garneinführungsvorrichtung 2 eingezeichnet, und zwar in einer Position, in welcher er die in der Prüfposition befindliche Garnprobe 9 ergreift. Das Ergreifen erfolgt zwischen den beiden Garnklemmen 82, 83. Dort ist die Position der Garnprobe 9 am besten definiert, und die Garnprobe 9 ist dort straff gespannt, während sie von den beiden Garnklemmen 82, 83 eingeklemmt ist. Nachdem der Greifer 5 die Garnprobe 9 ergriffen hat, werden die beiden Garnklemmen 82, 83 aktiv geöffnet, und die Garneinführungsvorrichtung 2 führt die ergriffene Garnprobe 9 über das Umlenkrad 86 bis zur Absaugöffnung 14 hin in den Garnpfad 12 ein.

Wie aus den Figuren 1 und 2 ersichtlich, ist die Garnwechselvorrichtung 6 auf einer Decke des Garnprüfgerätes 1 angebracht und gegenüber der Front 11 des Garnprüfgerätes 1 um mindestens einen Drittel und vorzugsweise um mehr als die Hälfte der Tiefe des Garnprüfgerätes 1 nach hinten versetzt. Dies erleichtert das Einlegen der Garnproben 9 von hinten.

**Figur 3** zeigt schematisch die gegenseitige Anordnung der Garnwechselvorrichtung 6 und der Garneinführungsvorrichtung 2 im erfindungsgemässen Garnprüfgerät 1; zum besseren Verständnis wird empfohlen, diese schematische Zeichnung zusammen mit der perspektivischen Ansicht von Figur 1 zu betrachten. Der hier als gerade angenommene Garnpfad 12 verläuft im Wesentlichen senkrecht nach unten in einer Richtung z. Die Front 11 des Garnprüfgerätes 1 definiert eine Frontebene 111, die durch die Achsen x und z aufgespannt wird und die den Garnpfad 12 enthält. Die Verschiebungsrichtung 75 der Einspanneinrichtung 8 liegt parallel zur Richtung x, d. h. senkrecht zum Garnpfad 12 und parallel zur Frontebene 111. Der Greifer 5 der Garneinführungsvorrichtung 2 ist einerseits in einer Richtung 101, die parallel zum Garnpfad 12 und zur Richtung z liegt, linear verschiebbar. Andererseits ist der Greifer 5 der Garneinführungsvorrichtung 2 um eine Drehachse 102, die senkrecht zum Garnpfad 12 und parallel zur Frontebene 111 liegt, drehbar, was mit einem Doppelpfeil 103 angedeutet ist. Man kann auch sagen, dass die Drehachse 102 parallel zur Verschiebungsrichtung 75 der Einspanneinrichtung 8 und zur Richtung x liegt. Die Drehebene, in welcher die Drehung 103 des Greifers 5 stattfindet, wird somit von den Achsen y und z aufgespannt und steht senkrecht auf der Frontebene 111.

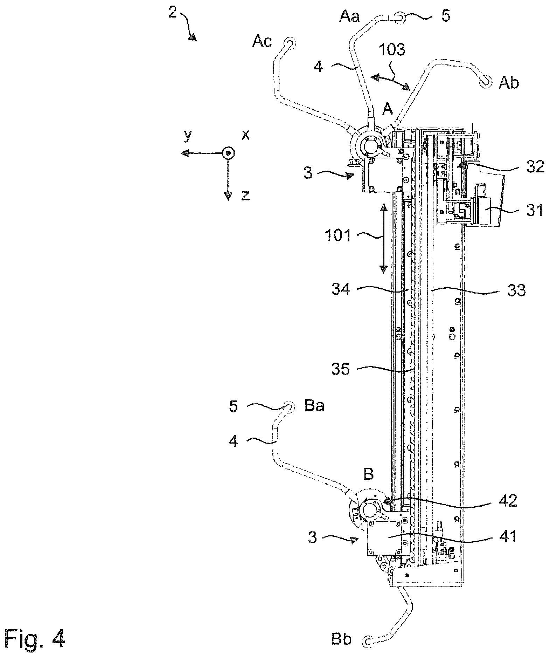

Eine Garneinführungsvorrichtung 2 für das erfindungsgemässe Garnprüfgerät 1 ist in **Figur 4** in einer Seitenansicht dargestellt, wobei bewegliche Elemente der Garneinführungsvorrichtung 2 in mehreren möglichen Positionen eingezeichnet sind. In dieser Ausführungsform beinhaltet die Garneinführungsvorrichtung 2 einen Wagen 3. Der Wagen 3 ist entlang einer Führungsschiene 34 linear hin und her verschiebbar, was mit einem Doppelpfeil 101 angedeutet ist. Die Richtung 101 der Linearverschiebung entspricht im Wesentlichen derjenigen des Garnpfades 12 und der Achse z (siehe Figuren 1 und 3). Der Wagen 3 ist auf einem endlosen Zahnriemen 33 befestigt und wird über den Zahnriemen 33 von einem im Garnprüfgerät 1 befestigten ersten Motor 31 zur Verschiebung angetrieben. Der Zahnriemen 33 ist über ein erstes Riemengetriebe 32 mit Untersetzung mit dem ersten Motor 31 verbunden. Der Wagen 3 ist in zwei verschiedenen Positionen A, B eingezeichnet. Der maximale Verschiebungsweg des Wagens 3 beträgt in diesem Ausführungsbeispiel ca. 60 cm.

Auf dem Wagen 3 ist ein Greifarm 4 drehbar gelagert; die Drehung ist mit einem Doppelpfeil 103 angedeutet. Eine Drehachse 102 der Drehung 103 liegt senkrecht zum Garnpfad 12 und parallel zur Frontebene 111 (siehe Figur 3). Zum Antrieb der Drehung ist auf dem Wagen 3 ein zweiter Motor 41 befestigt, der über ein zweites Riemengetriebe 42 mit Untersetzung mit dem Greifarm 4 verbunden ist. Der Greifarm 4 und die Greifeinrichtung 5 sind in fünf verschiedenen Positionen Aa, Ab, Ac, Ba, Bb eingezeichnet. Der maximale Drehwinkel des Greifarms 4 beträgt in diesem Ausführungsbeispiel ca. 240°.

Der erste Motor 31 und der zweite Motor 41 können individuell angesteuert werden. Die Verschiebung des Wagens 3 und die Drehung des Greifarms 4 sind also unabhängig voneinander. Eine Zuführung von elektrischen Kabeln und/oder Druckluftschläuchen zum Wagen 3 erfolgt über eine Schleppkette 35, die einerseits an einem Kabelabgang des Garnprüfgerätes 1 und andererseits am Wagen 3 befestigt ist.

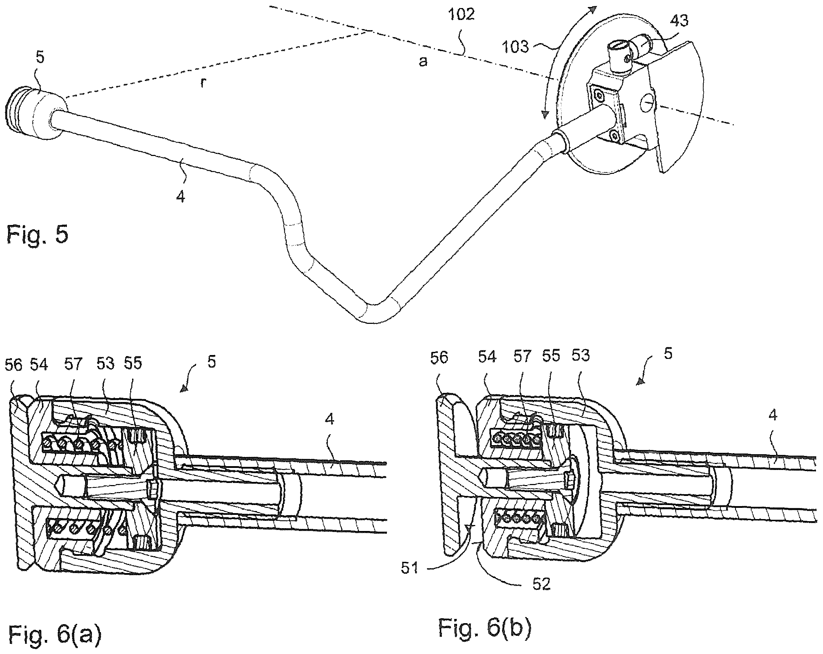

**Figur 5** zeigt einen Teil der Garneinführungsvorrichtung 2 von Figur 4 in einer perspektivischen Ansicht von links vorn. Der Greifarm 4 ist im dreidimensionalen Raum so geformt, dass er seine Funktion ausüben kann, ohne mit anderen Teilen des Garnprüfgerätes 1 zu kollidieren und dabei möglichst wenig aus dem Garnprüfgerät 1 heraus zu ragen. An einem freien Ende des Greifarms 4 befindet sich die Greifeinrichtung 5, auf die anlässlich der Figur 6 detailliert eingegangen wird. Der Greifarm 4 ist derart ausgebildet, dass die Greifeinrichtung 5 einerseits in axialer Richtung vom Wagen 3 und andererseits in radialer Richtung von der Drehachse 102 beabstandet ist. Die Abstände betragen z. B. a ≈ 16 cm in axialer Richtung vom Wagen 3 und r ≈ 20 cm in radialer Richtung von der Drehachse 102.

Die Greifeinrichtung 5 wird anhand der **Figur 6** erläutert. Um die Garnprobe 9 ergreifen und loslassen zu können, hat die Greifeinrichtung 5 einen geschlossenen Zustand, der in **Figur 6(a)** dargestellt ist, und einen offenen Zustand, der in **Figur 6(b)** dargestellt ist. Die beiden Zustände können reversibel ineinander übergeführt werden. Die Greifeinrichtung 5 ist im Wesentlichen als einfachwirkender Pneumatikzylinder mit einem Zylindergehäuse 53 ausgestaltet. In dem Zylindergehäuse 53 befindet sich ein axial verschiebbarer Kolben 55. Auf dem Kolben 55 ist ausserhalb des Zylindergehäuses 53 ein Klemmteller 56 befestigt, dessen innere, ebene Fläche als erste Klemmfläche 51 wirkt. Eine Decke 54 des Zylindergehäuses 53 bildet eine zweite Klemmfläche 52. Die erste Klemmfläche 51 und die zweite Klemmfläche 52 sind im Wesentlichen zueinander parallel und wirken zusammen, um im geschlossenen Zustand (Figur 6(a)) die Garnprobe 9 einzuklemmen. Die dazu benötigte Klemmkraft wird von einer zwischen dem Kolben 55 und der Decke 54 vorgespannten Druckfeder 57 ausgeübt, deren Federkraft die beiden Klemmflächen 51, 52 gegeneinander drückt.

Für die Öffnung der Greifeinrichtung 5 wird ihr durch den Greifarm 4, der als Rohr ausgebildet ist, über einen Lufteinlass 43 (siehe Figur 5) Druckluft zugeführt. Die zugeführte Druckluft drückt entgegen der Federkraft der Druckfeder 57 auf den Kolben 55 und entfernt so die beiden Klemmflächen 51, 52 voneinander. In diesem geöffneten Zustand (Figur 6(b)) lässt die Greifeinrichtung 5 die Garnprobe 9 los und ist danach bereit, erneut eine Garnprobe 9 zu ergreifen. Bei einer Verminderung des Luftdrucks schliesst sie sich wieder.

Statt einer aktiv betätigbaren Greifeinrichtung 5, von der eine Ausführungsform oben beschrieben ist, kann das erfindungsgemässe Garnprüfgerät 1 eine passive Greifeinrichtung wie z. B. eine Gabel beinhalten.

Nachfolgend wird die Funktionsweise des erfindungsgemässen Garnprüfgerätes 1 beschrieben.

Im Ruhezustand befindet sich der Greifarm in einer Ruheposition Aa (siehe Figur 4). Die in der Prüfposition befindlichen Garnklemmen 82, 83 sind geschlossen, und eine zuvor manuell eingelegte Garnprobe 9 ist zwischen den Garnklemmen 82, 83 eingespannt. Durch Zufuhr von Druckluft wird die Greifeinrichtung 5 geöffnet. Der Greifarm 4 wird um ca. -60° nach hinten gedreht, und so in eine Greifposition Ab gebracht, in welcher er auch in Figur 2 dargestellt ist. Die Druckluft wird abgestellt und die in der Prüfposition eingespannte Garnprobe 9 zwischen den beiden Klemmflächen 51, 52 der Greifeinrichtung 5 eingeklemmt. Nachdem die Greifeinrichtung 5 die Garnprobe 9 ergriffen hat, werden die beiden Garnklemmen 82, 83 geöffnet. Nun kann die Garnprobe 9 von der Garneinführungsvorrichtung 2 in den Garnpfad 12 eingeführt werden. Zu diesem Zweck wird der Greifarm 4, der die Garnprobe 9 hält, um ca. 100° nach vorn in eine erste Verschiebeposition Ac gedreht. Danach wird er um ca. 60 cm nach unten (in Richtung +z) in eine zweite Verschiebeposition Ba verschoben. Die Verschiebung wird beendet und der Greifarm 4 um weitere ca. 140° nach unten in eine Abgabeposition Bb gedreht. Das Ende der Garnprobe 9 befindet sich dann im Bereich der Absaugöffnung 14. Die Greifeinrichtung 5 wird erneut mit Druckluft beaufschlagt, so dass sie die Garnprobe 9 loslässt. Die Garnprobe 9 wird in die Absaugöffnung 14 hinein abgesaugt. Der Greifarm 4 wird um ca. -140° in die zweite Verschiebeposition Ba zurück gedreht, und die Greifeinrichtung 5 wird geschlossen. Danach wird der Greifarm 4 nach oben (in Richtung -z) in die erste Verschiebeposition Ac verschoben und um ca. -40° in die Ruheposition Aa gedreht. Dort verbleibt er während der Prüfung der Garnprobe 9. Im Betriebszustand, während die Garnprobe 9 von der Fördereinrichtung 13 durch den Garnpfad 12 gefördert und von den Sensoren 17 geprüft wird, müssen die beiden Garnklemmen 82, 83 offen bleiben, um den freien Lauf der Garnprobe 9 durch die Einspannstelle 8 nicht zu behindern.

Nach Beendigung der Prüfung wird die Fördereinrichtung 13 abgestellt, und die Garnprobe 9 steht still. Nun werden die Garnklemmen 82, 83 wieder geschlossen, so dass sie die Garnprobe 9 erneut einspannen. Der Greifarm 4 wird um ca. 9 cm nach unten (in Richtung +z) verschoben, so dass die Greifeinrichtung 5 die Garnprobe 9 nach unten, in die Schneideinrichtung 85 hinein, drückt. Die Schneideinrichtung 85 wird betätigt und schneidet die Garnprobe 9 stromabwärts der Einspannstelle 8 durch. Der Greifarm 4 wird um ca. 9 cm nach oben (in Richtung -z) in die Ruheposition Aa zurück verschoben Danach verschiebt die Transporteinrichtung 7 die Einspanneinrichtung 7 so weit, bis sich die nächste zu prüfende Garnprobe an der Prüfposition befindet; alternativ kann dieselbe Garnprobe 9 ohne Verschiebung der Einspanneinrichtung 7 nochmals geprüft werden. Sobald sich die nächste zu prüfende Garnprobe in der Prüfposition befindet, ist das Garnprüfgerät 1 bereit für einen neuen Prüfzyklus.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören.

### BEZUGSZEICHENLISTE

- 1: Prüfgerät
- 10: Deckplatte
- 11: Front
- 12: Garnpfad
- 13: Fördereinrichtung
- 14: Absaugöffnung
- 15, 16: Funktionsmodule
- 17: kapazitive Sensorbaugruppe

- 2: Garneinführungsvorrichtung

- 3: Wagen
- 31: erster Motor
- 32: erstes Riemengetriebe
- 33: Zahnriemen
- 34: Führungsschiene
- 35: Schleppkette

- 4: Greifarm
- 41: zweiter Motor
- 42: zewites Riemengetriebe

- 5: Greifeinrichtung
- 51,52: Klemmflächen
- 53: Zylindergehäuse
- 54: Decke
- 55: Kolben
- 56: Klemmteller
- 57: Druckfeder

- 6: Garnwechselvorrichtung

- 7: Einspanneinrichtung
- 70: Trägerplatte
- 71: längere Seite der Trägerplatte
- 72: kürzere Seite der Trägerplatte
- 73, 74: Flansche an der Trägerplatte
- 75: Halterung für Garnklemmen

- 8: Einspannstelle
- 80: Gerade
- 81: Garneinlauföse
- 82, 83: Garnklemmen
- 84: Garnauslauföse
- 85: Schneideinrichtung
- 86: Umlenkrad
- 9: Garnprobe
- 91: Laufrichtung der Garnprobe

- 101: Verschiebungsrichtung der Garneinführungsvorrichtung
- 102: Drehachse der Garneinführungsvorrichtung
- 103: Drehung der Garneinführungsvorrichtung
- 111: Frontebene

- A, B: Positionen des Wagens
- Aa, Ab, Ac, Ba, Bb: Positionen der Greifeinrichtung

## Patentansprüche

1. Garnprüfgerät (1) zur Prüfung einer Garnprobe (9), mit
einem entlang einer Front (11) des Garnprüfgerätes (1) verlaufenden Garnpfad (12), mindestens einem entlang des Garnpfades (12) angeordneten Sensor (17) zur Prüfung der Garnprobe (9) und
einer Garneinführungsvorrichtung (2) zur Einführung der Garnprobe (9) in den Garnpfad (12), welche Garneinführungsvorrichtung (2) eine bewegliche Greifeinrichtung (5) zum reversiblen Ergreifen der Garnprobe (9) aufweist, **dadurch gekennzeichnet, dass**
die Greifeinrichtung (5) einerseits in einer Richtung (101), die im Wesentlichen parallel zum Garnpfad (12) liegt, verschiebbar und andererseits um eine Drehachse (102), die senkrecht zum Garnpfad (12) und parallel zu einer durch die Front (11) definierten Frontebene (111) liegt, drehbar ist, wobei die Verschiebbarkeit und die Drehbarkeit unabhängig voneinander sind.

2. Garnprüfgerät (1) nach Anspruch 1, wobei die Garneinführungsvorrichtung (2) einen verschiebbaren Wagen (3) beinhaltet, auf dem ein Greifarm (4) drehbar gelagert ist, an dessen freiem Ende sich die Greifeinrichtung (5) befindet.

3. Garnprüfgerät (1) nach Anspruch 2, wobei der Wagen (3) linear verschiebbar ist.

4. Garnprüfgerät (1) nach Anspruch 2 oder 3, wobei der Wagen (3) von einem im Garnprüfgerät (1) befestigten ersten Motor (31) über mindestens einen Treibriemen (33) zur Verschiebung antreibbar ist.

5. Garnprüfgerät (1) nach einem der Ansprüche 2-4, wobei der Greifarm (4) von einem auf dem Wagen (3) befestigten zweiten Motor (41) zur Drehung antreibbar ist.

6. Garnprüfgerät (1) nach einem der Ansprüche 2-5, wobei der Greifarm (4) derart ausgebildet ist, dass die Greifeinrichtung (5) einerseits in axialer Richtung vom Wagen (3) und andererseits in radialer Richtung von der Drehachse (102) beabstandet ist.

7. Garnprüfgerät (1) nach einem der vorangehenden Ansprüche, wobei die Greifeinrichtung (5) pneumatisch betätigbar ist.

8. Garnprüfgerät (1) nach einem der Ansprüche 2-6 einerseits und nach Anspruch 7 andererseits, wobei der Greifarm (4) als Rohr ausgebildet ist, durch welches Druckluft der Greifeinrichtung (5) zuführbar ist.

9. Garnprüfgerät (1) nach einem der vorangehenden Ansprüche, wobei die Greifeinrichtung (5) ein Paar im Wesentlichen zueinander parallele, zusammenwirkende Klemmflächen (51, 52), einen Schliessmechanismus zum Drücken der Klemmflächen (51, 52) gegeneinander und einen Öffnungsmechanismus zum Entfernen der Klemmflächen (51, 52) voneinander aufweist.

10. Garnprüfgerät (1) nach Anspruch 7 oder 8 einerseits und nach Anspruch 9 andererseits, wobei der Schliessmechanismus eine vorgespannte Feder (57) beinhaltet, deren Federkraft die Klemmflächen (51, 52) gegeneinander drückt, und der Öffnungsmechanismus einen einfachwirkenden Pneumatikzylinder (53) beinhaltet, bei dessen Beaufschlagung mit Druckluft die Klemmflächen (51, 52) voneinander entfernt werden.

11. Garnprüfgerät (1) nach einem der vorangehenden Ansprüche, wobei das Garnprüfgerät (1) eine halbautomatische Garnwechselvorrichtung (6) zur Bereitstellung einer Mehrzahl von Garnproben (9) aufweist, welche halbautomatische Garnwechselvorrichtung (6)
eine Einspanneinrichtung (7) mit einer Mehrzahl von starr nebeneinander angeordneten, voneinander beabstandeten Einspannstellen (8) zum Einspannen der Garnproben (9) und
eine Transporteinrichtung zum Transportieren der Einspanneinrichtung (7), wodurch eine zu prüfende, an irgendeiner der Einspannstellen (8) eingespannte Garnprobe (9) in eine vorgegebene Prüfposition transportierbar ist,
beinhaltet, und
die Garnreinführungsvorrichtung (2) derart ausgestaltet und bezüglich der Garnwechselvorrichtung (6) angeordnet ist, dass eine in der Prüfposition eingespannte Garnprobe (9) von ihrer Greifeinrichtung (5) ergreifbar ist.

12. Garnprüfgerät (1) nach Anspruch 11, wobei jede Einspannstelle (8) eine Garneinlauföse (81) und zwei stromabwärts der Garneinlauföse (81) angeordnete, fluchtende Garnklemmen (82, 83) beinhaltet, und wobei die Garneinlauföse (81) und die beiden Garnklemmen (81, 82) voneinander beabstandet im Wesentlichen auf einer Geraden (80) liegen.

13. Garnprüfgerät (1) nach Anspruch 11 oder 12, wobei die halbautomatische Garnwechselvorrichtung (6) auf einer Decke des Garnprüfgerätes (1) angebracht und gegenüber der Front (11) des Garnprüfgerätes (1) um mindestens einen Drittel der Tiefe des Garnprüfgerätes (1) nach hinten versetzt ist.

14. Garnprüfgerät (1) nach einem der Ansprüche 11-13, wobei die Transporteinrichtung (7) der halbautomatischen Garnwechselvorrichtung (6) derart ausgestaltet ist, dass sie eine Linearverschiebung der Einspanneinrichtung (8) bewirkt und die Richtung (75) der Linearverschiebung senkrecht zum Garnpfad (12) und parallel zu der Frontebene (111) liegt.

## Claims

1. A yarn-testing device (1) for testing a yarn sample (9), comprising
a yarn path (12) extending along a front (11) of the yarn-testing device (1),
at least one sensor (17) arranged along the yarn path (12) for testing the yarn sample (9), and
a yarn insertion device (2) for inserting the yarn sample (9) into the yarn path (12), which yarn insertion device (2) comprises a movable gripping device (5) for reversibly gripping the yarn sample (9),
**characterized in that**
the yarn gripping device (5) is on the one hand displaceable in a direction (101) which lies substantially parallel to the yarn path (12) and is on the other hand rotatable about a rotational axis (102) which lies perpendicularly to the yarn path (12) and parallel to a front plane (111) which is defined by the front (11), wherein the displaceability and the rotatability are independent of each other.

2. A yarn-testing device (1) according to claim 1, wherein the yarn insertion device (2) comprises a displaceable carriage (3) on which a gripper arm (4) is rotatably mounted, at the free end of which the gripping device (5) is disposed.

3. A yarn-testing device (1) according to claim 2, wherein the carriage (3) is linearly displaceable.

4. A yarn-testing device (1) according to claim 2 or 3, wherein the carriage (3) is driveable for displacement by a first motor (31), which first motor (31) is fixed in the yarn-testing device (1), via at least one drive belt (33).

5. A yarn-testing device (1) according to one of the claims 2 to 4, wherein the gripper arm (4) is driveable for rotation by a second motor (41) fixed to the carriage (3).

6. A yarn-testing device (1) according to one of the claims 2 to 5, wherein the gripper arm (4) is formed in such a way that the gripping device (5) is spaced on the one hand in the axial direction from the carriage (3) and on the other hand in the radial direction from the rotational axis (102).

7. A yarn-testing device (1) according to one of the preceding claims, wherein the gripping device (5) is actuatable pneumatically.

8. A yarn-testing device (1) according to one of the claims 2 to 6 on the one hand and according to claim 7 on the other hand, wherein the gripper arm (4) is formed as a tube through which compressed air is suppliable to the gripping device (5).

9. A yarn-testing device (1) according to one of the preceding claims, wherein the gripping device (5) comprises a pair of substantially parallel, interacting clamping surfaces (51, 52), a closing mechanism for pressing the clamping surfaces (51, 52) against each other, and an opening mechanism for moving the clamping surfaces (51, 52) away from each other.

10. A yarn-testing device (1) according to claim 7 or 8 on the one hand and according to claim 9 on the other hand, wherein the closing mechanism contains a pretensioned spring (57) whose spring force presses the clamping surfaces (51, 52) against each other, and the opening mechanism contains a single-action pneumatic cylinder (53), by means of which the clamping surfaces (51, 52) are moveable away from each other when it is supplied with compressed air.

11. A yarn-testing device (1) according to one of the preceding claims, wherein
the yarn-testing device (1) comprises a semiautomatic yarn changing device (6) for providing a plurality of yarn samples (9), which semiautomatic yarn changing device (6) contains
a clamping device (7) with a plurality of mutually spaced clamping locations (8) which are rigidly arranged adjacent to each other for clamping the yarn samples (9), and
a transport device for transporting the clamping device (7), through which a yarn sample (9) which is to be tested and is clamped at any one of the clamping locations (8) is transportable to a predetermined testing position,
and the yarn insertion device (2) is formed and arranged with respect to the yarn changing device (6) in such a way that a yarn sample (9) clamped in the testing position is grippable by its gripping device (5).

12. A yarn-testing device (1) according to claim 11, wherein each clamping location (8) contains a yarn inlet eyelet (81) and two aligned yarn clamps (82, 83) which are arranged downstream of the yarn inlet eyelet (81), and wherein the yarn inlet eyelet (81) and the two yarn clamps (81, 82) lie substantially on a straight line (80) spaced from each other.

13. A yarn-testing device (1) according to claim 11 or 12, wherein the semiautomatic yarn changing device (6) is attached to a ceiling of the yarn-testing device (1) and offset to the rear in relation to the front (11) of the yarn-testing device (1) by at least one-third of the depth of the yarn-testing device (1).

14. A yarn-testing device (1) according to one of the claims 11 to 13, wherein the transport device (7) of the semiautomatic yarn changing device (6) is formed in such a way that it produces a linear displacement of the clamping device (8) and the direction (75) of the linear displacement lies perpendicularly to the yarn path (12) and parallel to the front plane (111).

## Revendications

1. Appareil de contrôle de fil (1) pour le contrôle d'un échantillon de fil (9), avec
un trajet de fil (12) passant le long d'une face avant (11) de l'appareil de contrôle de fil (1),
au moins un capteur (17) disposé le long du trajet de fil (12) pour contrôler l'échantillon de fil (9) et
un dispositif d'introduction de fil (2) pour l'introduction de l'échantillon de fil (9) dans le trajet de fil (12), lequel dispositif d'introduction de fil (2) présente un dispositif de préhension (5) mobile pour saisir de façon réversible l'échantillon de fil (9),
**caractérisé en ce que**
le dispositif de préhension (5) est capable, d'une part, de translation dans une direction (101) sensiblement parallèle au trajet de fil (12) et d'autre part de rotation autour d'un axe de rotation (102) perpendiculaire au trajet de fil (12) et parallèle à un plan frontal (111) défini par la face avant (11), la mobilité en translation et la mobilité en rotation étant indépendantes l'une de l'autre.

2. Appareil de contrôle de fil (1) selon la revendication 1, dans lequel le dispositif d'introduction de fil (2) comprend un chariot (3) capable de translation sur lequel un bras de préhension (4) à l'extrémité duquel se trouve le dispositif de préhension (5) est disposé avec possibilité de rotation.

3. Appareil de contrôle de fil (1) selon la revendication 2, dans lequel le chariot (3) est capable de translation linéaire.

4. Appareil de contrôle de fil (1) selon la revendication 2 ou 3, dans lequel le chariot (3) peut être entraîné en translation par un premier moteur (31) fixé dans l'appareil de contrôle de fil (1) par l'intermédiaire d'au moins une courroie d'entraînement (33).

5. Appareil de contrôle de fil (1) selon l'une des revendications 2 à 4, dans lequel le bras de préhension (4) peut être entraîné en rotation par un deuxième moteur (41) fixé sur le chariot (3).

6. Appareil de contrôle de fil (1) selon l'une des revendications 2 à 5, dans lequel le bras de préhension (4) est conformé de telle manière que le dispositif de préhension (5) est écarté, d'une part, du chariot (3) dans le sens axial et d'autre part de l'axe de rotation (102) dans le sens radial.

7. Appareil de contrôle de fil (1) selon l'une des revendications précédentes, dans lequel le dispositif de préhension (5) peut être actionné pneumatiquement.

8. Appareil de contrôle de fil (1) selon l'une des revendications 2 à 6, d'une part, et selon la revendication 7 d'autre part, dans lequel le bras de préhension (4) est conformé comme un tube à travers lequel de l'air comprimé peut être amené au dispositif de préhension (5).

9. Appareil de contrôle de fil (1) selon l'une des revendications précédentes, dans lequel le dispositif de préhension (5) présente une paire de surfaces de
serrage (51, 52) sensiblement parallèles et coopérant entre elles, un mécanisme de fermeture pour presser les surfaces de serrage (51, 52) l'une contre l'autre et un mécanisme d'ouverture pour éloigner les surfaces de serrage (51, 52) l'une de l'autre.

10. Appareil de contrôle de fil (1) selon la revendication 7 ou 8, d'une part, et selon la revendication 9 d'autre part, dans lequel le mécanisme de fermeture comprend un ressort précontraint (57) dont la force de ressort pousse les surfaces de
serrage (51, 52) l'une contre l'autre, et le mécanisme d'ouverture comprend un vérin pneumatique à simple action (53) qui écarte les surfaces de serrage (51, 52) l'une de l'autre lorsqu'il reçoit de l'air comprimé.

11. Appareil de contrôle de fil (1) selon l'une des revendications précédentes, dans lequel
l'appareil de contrôle de fil (1) comprend un dispositif de changement de fil semi-automatique (6) pour la fourniture de multiples échantillons de fil (9), lequel dispositif de changement de fil semi-automatique (6) comprend
un dispositif de serrage (7) avec de multiples points de serrage (8) disposés de manière fixe les uns à côté des autres et écartés les uns des autres pour serrer les échantillons de fil (9) et
un dispositif de transport pour transporter le dispositif de serrage (7), de sorte qu'un échantillon de fil (9) à contrôler serré dans n'importe lequel des points de serrage (8) peut être transporté dans une position de contrôle prédéterminée,
et le dispositif d'introduction de fil (2) est conformé et disposé par rapport au dispositif de changement de fil (6) de telle façon qu'un échantillon de fil (9) serré dans la position de contrôle puisse être saisi par son dispositif de préhension (5).

12. Appareil de contrôle de fil (1) selon la revendication 11, dans lequel chaque point de serrage (8) comprend un oeillet d'entrée du fil (81) et deux pinces à fil (82, 83) alignées disposés en aval de l'oeillet d'entrée du fil (81) et l'oeillet d'entrée du fil (81) et les deux pinces à fil (81, 82) se trouvent à distance l'un des autres sensiblement sur la même ligne droite (80).

13. Appareil de contrôle de fil (1) selon la revendication 11 ou 12, dans lequel le dispositif de changement de fil semi-automatique (6) est disposé sur un panneau supérieur de l'appareil de contrôle de fil (1) et décalé vers l'arrière par rapport à la face avant (11) de l'appareil de contrôle de fil (1) d'au moins un tiers de la profondeur de l'appareil de contrôle de fil (1).

14. Appareil de contrôle de fil (1) selon l'une des revendications 11 à 13, dans lequel le dispositif de transport (7) du dispositif de changement de fil semi-automatique (6) est conformé de façon à réaliser une translation linéaire du dispositif de serrage (8) et la direction (75) de la translation linéaire est perpendiculaire au trajet de fil (12) et parallèle au plan de la face avant (111).
